(19) Europäisches Patentamt
European Patent Office
Office européen des brevets

(11) Veröffentlichungsnummer: **0 099 031**
**A1**

# (12) EUROPÄISCHE PATENTANMELDUNG

(21) Anmeldenummer: 83106417.5

(22) Anmeldetag: 01.07.83

(51) Int. Cl.³: C 07 C 87/34
A 61 K 31/13

(30) Priorität: 10.07.82 DE 3225879

(43) Veröffentlichungstag der Anmeldung: 25.01.84 Patentblatt 84/4

(84) Benannte Vertragsstaaten: AT BE CH DE FR GB IT LI NL SE

(71) Anmelder: **BASF Aktiengesellschaft**
**Carl-Bosch-Strasse 38**
**D-6700 Ludwigshafen(DE)**

(72) Erfinder: **Himmele, Walter, Dr.**
**Eichenweg 14**
**D-6909 Walldorf(DE)**

(72) Erfinder: **Heberle, Eolfgang, Dr.**
**Uhdestrasse 2b**
**D-8130 Starnberg(DE)**

(72) Erfinder: **Kohlmann, Friedrich-Wilhelm, Dr.**
**An der Duene 6**
**D-2082 Moorrege(DE)**

(72) Erfinder: **Wesenberg, Walter**
**Dorfstrasse 16**
**D-2421 Bujendorf ueber Eutin(DE)**

## (54) Trans-3-(4'-tert.-Butylcyclohexyl-1')-2-methyl-1-dialkylaminopropane, ihre Herstellung und Verwendung als Arzneimittel.

(57) Es werden trans-3-(4'-tert.-Butylcyclohexyl-1')-2-methyl-1-dialkylaminopropane der Formel I hergestellt,

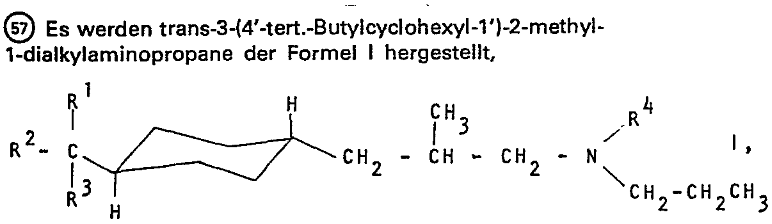

I,

worin $R^1$ und $R^2$ Alkylreste darstellen, wobei die Summe der C-Atome in $R^1$ und $R^2$ 5, 6 oder 7 ist, sowie deren Salze mit physiologisch verträglichen Säuren und deren Herstellung beschrieben. Die neuen Verbindungen eignen sich als Wirkstoffe für Arzneimittel.

EP 0 099 031 A1

Croydon Printing Company Ltd.

Trans-3-(4'-tert.-Butylcyclohexyl-1')-2-methyl-1-dialkylaminopropane, ihre Herstellung und Verwendung als Arzneimittel

Die Erfindung betrifft neue trans-3-(4'-tert.-Butylcyclohexyl-1')-2-methyl-1-dialkylaminopropane, Verfahren zu deren Herstellung sowie deren Verwendung bei der Bekämpfung von Krankheiten.

Es ist bereits bekannt, daß sich 3-(4'-tert.-Butylcyclohexyl-1')-2-methyl-1-(2',6'-dimethylmorpholino)-propan und dessen Salze zur Bekämpfung von pflanzenpathogenen Pilzen und von humanpathogenen Pilzen und Hefen eignen (DE-OS 27 52 135). Aus der DE-OS 29 21 221 ergibt sich, daß von diesen Verbindungen die Isomeren besonders wirksam sind, in denen die Substituenten am Cyclohexanring in bis-äquatorialer Stellung vorliegen. Die Abtrennung der bis-äquatorialen Verbindungen aus dem Stereoisomeren-Gemischen erfolgt durch Anreicherung mittels fraktionierter Destillation und anschließende Umkristallisation nach Überführen in ein Salz. Diese Trennung ist recht schwierig und aufwendig. So ist für die Destillation eine Temperatur erforderlich, bei der - insbesondere in Metallgefäßen - partielle Isomerisierungen am Morpholinrest auftreten, die die Wirkung der Substanzen herabsetzen.

Es wurden nun Verbindungen gefunden, die ebenfalls antimykotisch wirksam und darüber hinaus leichter herstellbar sind.

Gegenstand der Erfindung sind trans-3-(4'-tert.-Butylcyclohexyl-1')-2-methyl-1-dialkylaminopropane der Formel I

WK/P

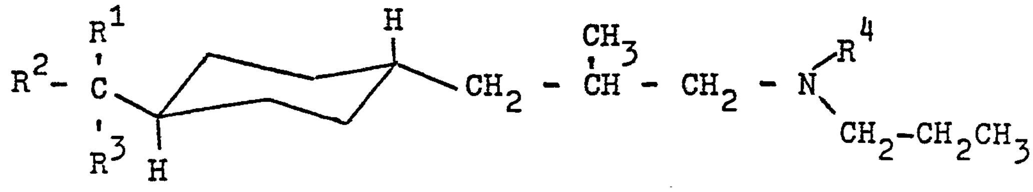 I,

worin

$R^1$, $R^2$ und $R^3$ gleich oder verschieden sind und $C_{1-4}$-Alkylreste darstellen, wobei auch zwei der Reste $R^1$, $R^2$ und $R^3$ zusammen einen Cyclopentyl- oder Cyclohexylrest bilden können,

und

$R^4$ einen Ethyl- oder n-Propylrest ist, sowie deren Salze mit physiologisch verträglichen Säuren.

In den trans-Verbindungen liegen die Substituenten am Cxclohexylring in der äquatorialen Stellung.

Als Reste $R^1$, $R^2$ und $R^3$ kommen insbesondere Methylgruppen in Betracht. $R^4$ ist vorzugsweise n-Propyl. Als physiologisch verträgliche Säuren sind beispielsweise organische Carbonsäuren oder anorganische Säuren, wie Salpetersäure, Salzsäure, Schwefelsäure, Phosphorsäure, Essigsäure, Propionsäure, Milchsäure, Bernsteinsäure, Weinsäure, Zitronensäure, Benzoesäure, Salicylsäure oder Nicotinsäure zu nennen. Bevorzugt sind jedoch die genannten anorganischen Säuren, insbesondere Salzsäure.

Die neuen Verbindungen werden hergestellt, indem man Verbindungen der Formel II

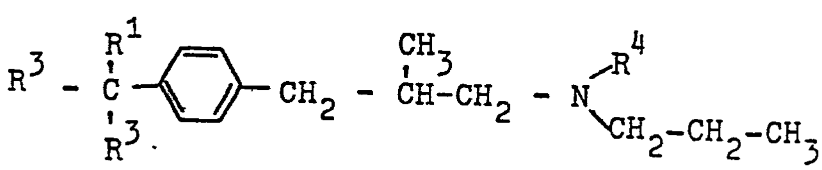 II,

worin $R^1$, $R^2$, $R^3$ und $R^4$ dasselbe wie oben bedeuten, hydriert, das so erhaltene Isomerengemisch in die Isomeren trennt und die so erhaltenen Verbindungen gegebenenfalls in ihre Salze mit physiologisch verträglichen Säuren überführt.

Die Hydrierung der Verbindungen II erfolgt am günstigsten bei 20 - 200°C und einem Wasserstoffdruck von 1 - 300 bar. Als Katalysatoren eignen sich besonders Ruthenium und Palladium. Die Hydrierung erfolgt zweckmäßigerweise in einem Lösungsmittel wie Dioxan, Isopropanol oder Eisessig. Es empfiehlt sich, die Wasserstoffaufnahme mit Hilfe des Protonenresonanzspektrums zu kontrollieren. Ist die Wasserstoffaufnahme komplett, wird das Reaktionsgemisch über eine Kolonne mit etwa 40 - 70 theoretische Trennstufen bei 10 - 40 mbar und einem Rücklaufverhältnis von 1 : 1 bis 1 : 20 fraktioniert destilliert. Die Destillation liefert die gewünschten Produkte in einer solchen Reinheit (über 98,5 %), daß sie direkt zur Herstellung der galenischen Zubereitungsform eingesetzt werden können. Die erfindungsgemäßen Verbindungen, bei denen die Substituenten in der Stellung äquatorial-äquatorial am Cyclohexylrest stehen, sieden höher als die entsprechenden in der äquatorial-axial-Stellung.

Die neuen Verbindungen sind somit wesentlich einfacher herstellbar als die in der DE-OS 29 21 221 genannten. Die destillative Trennung braucht nicht in einem so starken Vakuum zu erfolgen, so daß kleinere Apparaturen verwendet werden können. Bei der Destillation werden die Isomeren sauber getrennt und es treten nur kleine Übergangsfraktionen auf. Außerdem kann die Destillation schneller durchgeführt werden.

Die neuen Verbindungen und ihre Salze weisen starke antimykotische Wirkungen auf. Sie besitzen ein breites antimykotisches Wirkungsspektrum, insbesondere gegen Dermatophyten, wie z.B. Species der Gattungen Epidermophyton, Microsporum und Trichophyton und Hefen, wie z.B. Species der Gattung Candida. Die Aufzählung dieser Mikroorganismen soll keine Beschränkung der zu bekämpfenden Mikroorganismen darstellen, sondern nur zur Erläuterung dienen.

Die Wirkung gegenüber Dermatophyten kann nach Methoden, wie sie beispielsweise in P. Klein, Bakteriologische Grundlagen der chemotherapeutischen Laboratoriumspraxis, Springer-Verlag Berlin, Göttingen, Heidelberg, 1957, beschrieben sind, aufgezeigt werden. Die überraschende Wirkung gegenüber Hefen wurde im Pseudomyzel- und Myzelphasentest mit Candida albicans nachgewiesen. Die antimikrobielle Wirksamkeit in vitro gegen Mikroorganismen wurde im Agrar-Dilutionstest (P. Klein: loc. cit.) ermittelt.

Das Ergebnis zeigt Tabelle 1.

Ähnliche Ergebnisse erhält man mit vielen weiteren Stämmen der in der Tabelle 1 aufgeführten Arten sowie mit Stämmen der Arten Mikrosporon audouinii, Mikrosporon canis, Mikrosporon gypseum, Trichophyton violaceum, Trichophyton rubrum und Trichphyton quinckeanum.

Tabelle 1 : Minimale Hemmkonzentrationen

| Substanz des Beispiels | Trichophyton mentagroph. ATCC 4807 | Candida albicans ATCC 14052 | Epidermophyton floccosum ATCC 10227 | Mikrosporon ferrugineum CBS 317.31 |
|---|---|---|---|---|
| 1 | < 0,125 | <0,125 | <0,125 | <0,125 |
| 2 | 0,25 | 1 | 0,25 | <0,125 |

Weiter wurden im Modell der Meerschweinchentrichophytie (Trichophyton mentagrophytes) (vgl. Heffter-Heubner: Handbuch der experimentellen Pharmakologie, Band XVI/11A, Springer Verlag, Berlin, Heidelberg, New York, 1967) die neuen Verbindungen bei äußerlicher Anwendung in Form von 0,2-, 0,5-und 1%igen Suspension getestet. Die Applikation erfolgt einmal täglich 4 bzw. 7 Tage lang. Nach dem 4. und 7. Behandlungstag und 7 Tage nach der letzten Behandlung werden bei jedem Tier an 3 bis 5 Stellen des infizierten Gebietes Haare oder Schuppen gezupft und in Sabourand-Dextrose-Bouillon bebrütet.

Die Auswertung erfolgt anhand des Nachweises eines Pilzwachstums nach 8-tägiger Bebrütung.

Die Tabelle 2 zeigt, bei welchem Bruchteil der Tiere während und nach der Behandlung noch Infektionen beobachtet wurden.

Tabelle 2

| Substanz | | 4 Tage | 7 Tage | 7 Tage nach Behandlung |
|---|---|---|---|---|
| Beispiel 1 | 0,2 % | 3/5 | 2/5 | 1/5 |
| " | 0,5 % | 0/5 | 0/5 | 0/5 |
| " | 1,0 % | 1/5 | 0/5 | 0/5 |
| Clotrimazol | 0,2 % | 3/5 | 3/5 | 3/5 |
| Clotrimazol | 1,0 % | 2/5 | 0/5 | 3/5 |
| Tolnaftat | 1,0 % | 0/9 | 0/9 | 2/9 |
| Kontrolle | - | 5/5 | 5/5 | 5/5 |

Die Tabelle 2 zeigt die Überlegenheit der neuen Substanzen gegenüber den Vergleichssubstanzen.

Danach eignen sich die neuen Substanzen insbesondere zur Behandlung von Dermatomykosen, Dermatophytosen und Systemmykosen, insbesondere verursacht durch Species der Gattungen Epidermophyton, Microsporum und Trichophyton und Hefen, wie Species der Gattung Candida.

Die neuen Verbindungen können in den gebräuchlichen galenischen Applikationsformen als Lösungen, Puder, Salben, Cremes oder Sprays angewendet werden. Diese werden in üblicher Weise hergestellt. Die Wirkstoffe können dabei mit den üblichen galenischen Hilfsmitteln verarbeitet werden (vgl. H. Sucker et al: Pharmazeutische Technologie, Thieme-Verlag, Stuttgart, 1978).

In der Regel werden die Wirkstoffe in Mengen von etwa 0,07 bis 0,1 $g/cm^2$ Körperoberfläche je 24 Stunden, gegebenenfalls in Form mehrerer Einzelgaben, äußerlich zur Anwendung gebracht. Es kann jedoch auch von den genannten Dosierungen abgewichen werden, und zwar in Abhängigkeit von der Art und der Schwere der Erkrankung. Die Festlegung der jeweils erforderlichen optimalen Dosierung und deren Häufigkeit kann durch den behandelnden Arzt aufgrund seines Wissens variiert werden.

In der Regel werden die Einzelgaben 1- bis 2mal täglich aufgebracht. Bei äußerlicher, lokaler Anwendung enthalten die Zubereitungen 0,2 bis 1,0, bevorzugt 0,5 Gew.-% Wirkstoff.

Beispiel 1

A. Herstellung des Ausgangsmaterials

Zu 1428 g 3-(4'-tert.-Butylphenyl)-2-methylpropanal und 707 g Di-n-propylamin wurden unter Rühren im Verlauf von 19 h 401 g 98%ige Ameisensäure so zugegeben, daß keine starke Schaumbildung auftrat. Das entstandene $CO_2$ entwich über einen Rückflußkühler. Das so erhaltene Reaktionsgemisch wurde einer Kurzwegdestillation unterworfen, wobei man 1950 g Destillat erhielt, welches bei 110-180°C/0,6 mbar überging. Dieses wurde über eine Kolonne fraktioniert destilliert. Die bei 0,4 mbar und bis 136°C übergehende Fraktion bestand aus 97%igem 3-(4'-tert.-Butylphenyl)-2-methyl-1-di-n-propylaminopropan. Die Ausbeute betrug 1890 g.

B. Herstellung und Isolierung des Endprodukts

a) 1050 g des gemäß A erhaltenen Produkts wurden in 2000 ml Isopropanol in einem Autoklaven bei 100-140°C und einem Wasserstoffdruck von 100-140 bar unter Rühren hydriert. Als Katalysator diente 1 g Rutheniumoxidhydrat. Nach 44 h wurde das Reaktionsprodukt mit 25 g Aktivkohle gerührt und filtriert. Das Filtrat wurde eingeengt und anschließend einer Kurzwegdestillation unterworfen.

Das bei 125-135°C/0,6 mbar übergehende Produkt (1020 g) wurde an einer Kolonne fraktioniert destilliert.

b) Das gemäß a) erhaltene Produkt, das aus 56,2 % cis- und 43,8 % trans-1,4-Cyclohexanderivat bestand, wurde in einer mit Edelstahldrahtnetzwendeln gefüllten Kolonne (Höhe: 160 cm; Weite: 2,9 cm) bei einem Druck von 37 bar fraktioniert. Am Kolonnenkopf wurde ein Ab-

lauf-Rücklauf-Verhältnis von 1:10 eingestellt. Bis zu einer Temperatur von 195°C erhielt man 439 g eines Produktes, welches zu 96,5 % aus dem cis-Derivat bestand. Bei 195 - 198°C destillierten 331 g einer Übergangsfraktion über, die 47,2 % cis- und 52,7 % trans-Derivat enthielt. Bei 198°C ging anschließend trans-3-(4'-tert.-Butylcyclohexyl-1')-2-methyl-1-(N-di-n-propylamino)-propan über. Die Destillation wurde ohne Kolonnenkopf zu Ende geführt. Das Produkt (250 g) war zu 99,8 % rein.

Durch Wiedereinsetzen der Übergangsfraktion in die nächsten Destillationsansätze ließ sich die Ausbeute auf 96,5 % steigern.

Wegen der geringen Unterschiede in den Siedepunkten der cis- und trans-Verbindung muß die Destillation gaschromatographisch überwacht werden.

C. Überführen in ein Salz.

29,5 der gemäß B.b) erhaltenen trans-Verbindung wurden in 65 ml heißem Ethanol mit Fumarsäure versetzt. Beim Abkühlen kristallisierten 29 g Hydrogenfumarat aus, Fp. = 127-132°C. In ähnlicher Weise erhält man das Hydrobromid (Fp. = 141 - 147°C), das Hydroiodid (Fp. = 142 - 146°C) und das Oxalat (Fp. = 147°C).

Analog wurden hergestellt bzw. lassen sich herstellen:

2. trans-3-(4'-tert.-Butylcyclohexyl-1')-2-methyl-1-(N-ethyl-N-n-propylamino)-propan, Kp: 187 /33 mbar

3. trans-3-[4'-(1'-Dimethyl-n-propyl)-cyclohexyl-1')]-2-methyl-1-(N-ethyl-N-n-propylamino)-propan.

4. trans-3-[4'-(1"-Methylcyclopentyl-1")-cyclohexyl-1')]-methyl-(N-di-n-propylamino)-propan.

Beispiele für pharmazeutische Zubereitungen

I Creme

| | | | |
|---|---|---|---|
| a) | Wirkstoff (Beispiel 1, A.b)) | | 0,5 g |
| b) | Glycerinmonostearat | | 10,0 g |
| c) | Cetylalkohol | | 5,0 g |
| d) | Polyethylenglykol-400-stearat | | 10,0 g |
| e) | Polyethylenglykol-sorbitan-monostearat | | 10,0 g |
| f) | Propylenglykol | | 6,0 g |
| g) | p-Hydroxybenzoesäuremethylester | | 0,2 g |
| h) | Demineralisiertes Wasser | ad. | 100,0 g |

Der feinst gepulverte Wirkstoff wird im Propylenglykol suspendiert und die Suspension in die auf 65°C erwärmte Schmelze aus Glycerinmonostearat, Cetylalkohol, Polyethylenglykol-400-stearat und Polyethylenglykol-sorbitan-monostearat gerührt. In diese Mischung wird die 70°C heiße Lösung des p-Hydroxybenzoesäuremethylesters in Wasser emulgiert. Nach dem Erkalten wird die Creme über eine Kolloidmühle homogenisiert und in Tuben abgefüllt.

II Puder

| | | |
|---|---|---|
| a) | Wirkstoff (Beispiel 1, C) | 0,5 g |
| b) | Zinkoxid | 10,0 g |
| c) | Magnesiumoxid | 10,0 g |
| d) | Hochdisperses Siliciumoxid | 2,5 g |
| e) | Magnesiumstearat | 1,0 g |
| f) | Talkum | 75,5 g |

Der Wirkstoff wird auf einer Luftstrahlmühle mikronisiert und mit den anderen Bestandteilen homogen vermischt.

Die Mischung wird durch ein Sieb Nr. 7 geschlagen und in Polyethylenbehälter mit Streueinsatz abgefüllt.

Patentansprüche

1. Trans-3-(4'-tert.-Butylcyclohexyl-1')-2-methyl-1-dialkylaminopropane der Formel I

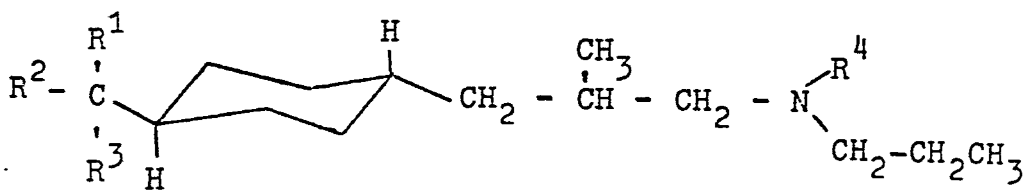

I,

worin

$R^1$, $R^2$ und $R^3$ gleich oder verschieden sind und $C_{1-4}$-Alkylreste darstellen, wobei auch zwei der Reste $R^1$, $R^2$ und $R^3$ zusammen einen Cyclopentyl- oder Cyclohexylrest bilden können,
und

$R^4$ einen Ethyl- oder n-Propylrest ist, sowie deren Salze mit physiologisch verträglichen Säuren.

2. Trans-3-(4'-tert.-Butylcyclohexyl-1')-2-methyl-1-(N--di-n-propylamino)-propan.

3. Trans-3-(4'-tert.-Butylcyclohexyl-1')-2-methyl-1-(N--ethyl-N-n-propylamino)-propan.

4. Verfahren zur Herstellung der Verbindungen der Formel 1, dadurch gekennzeichnet, daß man Verbindungen der Formel II

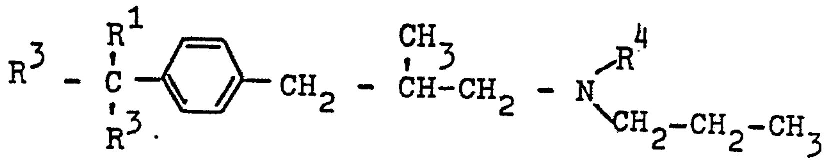 II,

worin $R^1$ und $R^2$ dasselbe wie oben bedeuten, hydriert, das so erhaltene Isomerengemisch in die Isomeren trennt und die so erhaltenen Verbindungen gegebenenfalls in ihre Salze mit physiologisch verträglichen Säuren überführt.

5. Arzneimittel, enthaltend eine Verbindung gemäß Anspruch 1.

6. Verbindung der Formel I gemäß Anspruch 1 zur Verwendung bei der Bekämpfung von Krankheiten.

Patentansprüche (für Österreich)

1. Verfahren zur Herstellung von trans-3-(4'-tert.-Butylcyclohexyl-1')-2-methyl-1-dialkylaminopropanen der Formel I

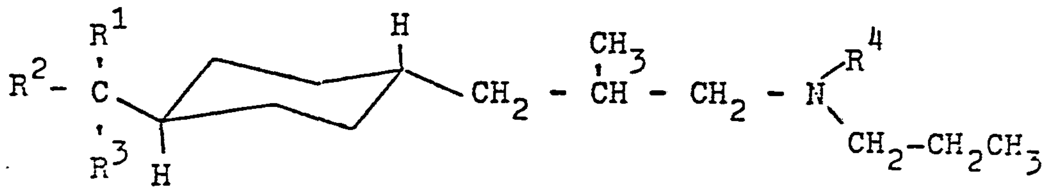

I,

worin

$R^1$, $R^2$ und $R^3$ gleich oder verschieden sind und $C_{1-4}$-Alkylreste darstellen, wobei auch zwei der Reste $R^1$, $R^2$ und $R^3$ zusammen einen Cyclopentyl- oder Cyclohexylrest bilden können, und

$R^4$ ein Ethyl- oder n-Propylrest ist, sowie deren Salze mit physiologisch verträglichen Säuren,

dadurch gekennzeichnet, daß man Verbindungen der Formel II

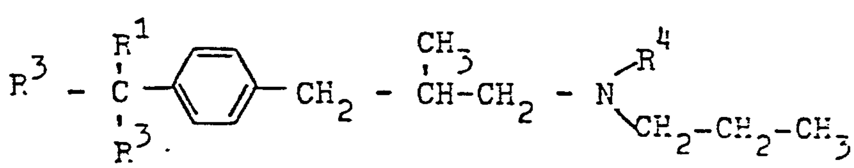

II,

worin $R^1$ und $R^2$ dasselbe wie oben bedeuten, hydriert, das so erhaltene Isomerengemisch in die Isomeren trennt und die so erhaltenen Verbindungen gegebenenfalls in ihre Salze mit physiologisch verträglichen Säuren überführt.

2. Verfahren gemäß Anspruch 1, dadurch gekennzeichnet, daß man trans-3-(4'-tert.-Butylcyclohexyl-1')-2-

-methyl-1-(N-di-n-propylamino)-propan herstellt.

3. Verfahren gemäß Anspruch 1, <u>dadurch gekennzeichnet</u>, daß man trans-3-(4'-tert.-Butylcyclohexyl-1')-2-methyl-1-(N-ethyl-N-n-propylamino)-propan herstellt.

0099031

Europäisches Patentamt

# EUROPÄISCHER RECHERCHENBERICHT

Nummer der Anmeldung

EP 83 10 6417

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | EP-A-0 019 764 (BASF) * Seite 1, Zeile 20 - Seite 2, Zeile 7 * & DE-A1-2 921 221 (Cat. D) | 1-6 | C 07 C 87/34<br>A 61 K 31/13 |
| | --- | | |
| A | CH-A- 237 879 (LES LABORATOIRES FRANCAIS DE CHIMIOTHERAPIE) * Anspruch; Spalte 2, Zeilen 37-39 * | 1-6 | |
| | ----- | | |
| | | | **RECHERCHIERTE SACHGEBIETE (Int. Cl. 3)** |
| | | | C 07 C 87/00 |

Der vorliegende Recherchenbericht wurde für alle Patentansprüche erstellt.

| Recherchenort | Abschlußdatum der Recherche | Prüfer |
|---|---|---|
| BERLIN | 08-09-1983 | BREW C.H. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82